# EUROPEAN PATENT APPLICATION

(11) **EP 4 729 088 A1**
(43) Date of publication of application: **22.04.2026**
(21) Application number: 24207538.0
(22) Date of filing: 18.10.2024
(51) Int. Cl.: A61M 5/142, A61M 5/145

(54) **POWER DEVICE AND PATCH TYPE INSULIN PUMP**

(71) Applicant: Syai UK Ltd, Cambridge CB2 8DL (GB)
(72) Inventor: WANG, Zhihua, Cambridge, CB2 8DL (GB)
(74) Representative: Nederlandsch Octrooibureau

(57) **Abstract**

The present application relates to the technical field of medical instruments, and disclosed thereby are a power device and a patch type insulin pump. The power device comprises a power output module, a transmission component, a lead screw and a sliding block, wherein the power output module comprises a power output shaft, and the power output shaft is in transmission connection to the transmission component. The transmission component is in transmission connection to the lead screw, and the transmission component is used to transfer power output from the power output shaft to the lead screw to drive the lead screw to rotate around its own axis. The sliding block is sleeved on the lead screw, and the sliding block is abutted to one end of a push rod away from a bolus piston. When the lead screw rotates around its own axis, the sliding block is driven to move relative to the lead screw along the axis direction of the lead screw, so that the sliding block drives the push rod to move along the axis direction of the lead screw. The axis of the power output shaft is parallel to the axis of the lead screw, and the power output direction of the power output shaft is opposite to the movement direction of the sliding block. The power device disclosed by the application is beneficial for reducing the volume of the patch type insulin pump.

## Description

### Technical Field

The present application relates to the technical field of medical instruments, and particularly to a power device and a patch type insulin pump.

### Background

At present, the patch type insulin pump has achieved fully automatic bolus and drug injection. In order to achieve automatic bolus and drug injection, the power device used for bolus and drug injection in the patch type insulin pump usually comprises a power output module, a transmission module, and a driving module provided in order along the bolus direction. The power output module transmits the power to the transmission module, which in turn transmits it to the driving module. The driving module is used to achieve bolus and drug injection. However, due to the limitations of the power device structure mentioned above, the overall volume of the patch type insulin pump is too large, which in turn affects the user experience.

### Summary

The present application provides a power device and a patch type insulin pump, which can reduce the produce volume while achieving bolus and drug injection.

In a first aspect, the present application provides a power device, applied to a patch type insulin pump, wherein the patch type insulin pump comprises a cartridge, a bolus piston and a push rod; the cartridge is used to internally store medicament, and the bolus piston can move relative to the cartridge, in order to compress the space inside the cartridge; the push rod is connected to one side of the bolus piston away from the space inside the cartridge; the power device comprises a power output module, a transmission component, a lead screw, and a sliding block;
the power output module comprises a power output shaft, and the power output shaft is in transmission connection to the transmission component;
the transmission component is in transmission connection to the lead screw, and the transmission component is used to transfer power output from the power output shaft to the lead screw to drive the lead screw to rotate around its own axis;
the sliding block is sleeved on the lead screw, and the sliding block is abutted to one end of a push rod away from a bolus piston. When the lead screw rotates around its own axis, the sliding block is driven to move relative to the lead screw along the axis direction of the lead screw, so that the sliding block drives the push rod to move along the axis direction of the lead screw;
wherein, the axis of the power output shaft is parallel to the axis of the lead screw, and the power output direction of the power output shaft is opposite to the movement direction of the sliding block.

The power device provided by the present application transfers power output from the power output module to the lead screw through the transmission component to drive the lead screw to rotate around its own axis, thereby driving the sliding block to move relative to the lead screw along the axis direction of the lead screw, in order to push the bolus piston to move and achieve the bolus and drug injection of the patch type insulin pump. Since the axis of the power output shaft is parallel to the axis of the lead screw, and the power output direction of the power output shaft is opposite to the movement direction of the sliding block, so that the transmission path of the power output from the power output shaft is U-shaped. In other words, the power output module and the lead screw are structurally superposed in parallel, which can effectively shorten the length space of the power device in the movement direction of the bolus piston, thereby facilitating the reduction of the volume of the patch type insulin pump.

In some possible embodiments, the power output module comprises a stepping motor.

In some possible embodiments, the power device further comprises a reduction box, and the reduction box is connected between the power output shaft and the transmission component.

In some possible embodiments, the transmission component comprises at least two gears, and the at least two gears are meshed in order along the arrangement direction of the power output shaft and the lead screw; the reduction box and the lead screw are respectively in transmission connection to the two gears at both ends.

In some possible embodiments, the power device further comprises a support, and the power output module, the transmission component and the lead screw are installed on the support.

In some possible embodiments, the support comprises a first connecting plate, a second connecting plate and a third connecting plate; the first connecting plate and the second connecting plate are in parallel and spaced apart, and the third connecting plate is connected between the first connecting plate and the second connecting plate, so that the support is U-shaped;
the power output module is installed on one side of the third connecting plate away from the first connecting plate, and both ends of the lead screw are respectively connected to the first connecting plate and the second connecting plate.; the sliding block is located between the first connecting plate and the second connecting plate.

In some possible embodiments, the transmission component is installed on one side of the first connecting plate away from the second connecting plate; one end of the lead screw passes through the first connecting plate and in transmission connection to the transmission component; a portion of the transmission component protrudes from one end of the first connecting plate near the third connecting plate.

In some possible embodiments, the power device further comprises a guide plate; the guide plate is connected between the first connecting plate and the second connecting plate, and one side of the sliding block is in contact with the guide plate.

In some possible embodiments, the forward projection of the cartridge on a first plane covers the forward projection of the power device on the first plane, and the first plane is a plane perpendicular to the axis of the lead screw.

In a second aspect, the present application provides a patch type insulin pump, comprising the power device according to any of possible embodiments in the first aspect.

### Brief Description of the Drawings

Fig. 1 is a schematic diagram of the overall structure of connecting the power device to the bolus piston in the embodiment of the present application; and
Fig. 2 is a schematic diagram of the explosive structure connecting the power device to the bolus piston in Fig. 1.

In the drawings:
10-cartridge; 20-bolus piston; 30-push rod; 100-power output module; 110-stepping motor; 200-transmission component; 210, 210a, 210b, 210c-gear; 300-lead screw; 400-sliding block; 500-reduction box; 600-support; 610-first connecting plate; 620-second connecting plate; 621-avoidance hole; 630-third connecting plate; 700-guide plate.

### Detailed Description of the Preferred Embodiments

The technical solutions in the embodiments of the present application will be described clearly and completely in connection with the drawings in the embodiments of the present application in the following paragraphs. Obviously, the embodiments described are only some, not all, of the embodiments of the present application. All other embodiments obtained by those of ordinary skill in the art without creative work based on the embodiments of the present application are within the scope of protection of the present application.

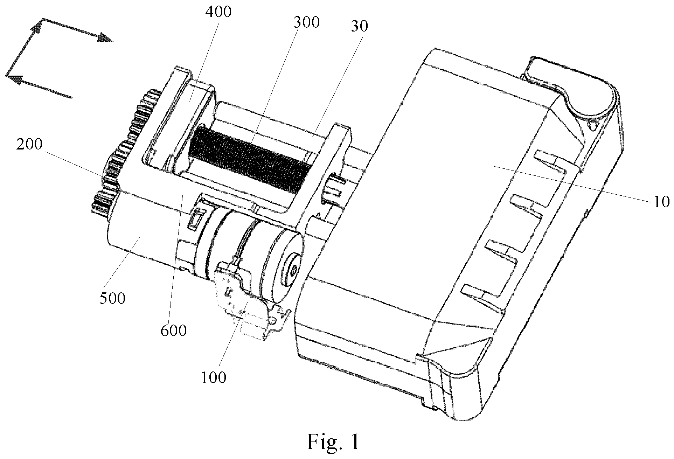

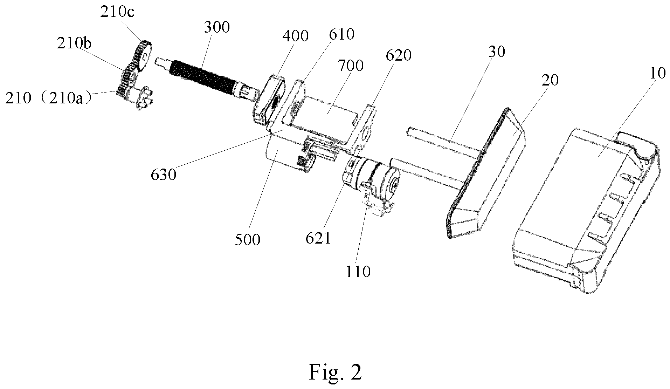

Referring to Fig. 1 and Fig. 2, the power device in the present application can be applied to the patch type insulin pump, in order to achieve bolus and drug injection by the patch type insulin pump. Specifically, the patch type insulin pump in this embodiment may comprise a cartridge 10, a bolus piston 20 and a push rod 30; there is a space inside the cartridge 10 for storing medicament, and one end of the cartridge 10 is provided with an opening; the bolus piston 20 is covered at the opening, and in tight fitting to the opening. The bolus piston 20 can move relative to the cartridge 10 to compress the space inside the cartridge 10, thereby allowing the medicament stored in the cartridge 10 to flow into the catheter and be injected into the user's body. The push rod 30 is connected to one side of the bolus piston 20 away from the space inside the cartridge 10. The extension direction of the push rod 30 is consistent with the movement direction of the bolus piston 20. The power device can act on the push rod 30 to transfer power to the bolus piston 20 through the push rod 30, achieving the movement of the bolus piston 20.

The power device may comprise a power output module 100, a transmission component 200, a lead screw 300, and a sliding block 400. The power output module 100 may comprise a power output shaft (not shown in the figure), and the power output shaft is in transmission connection to the transmission component 200. The transmission component 200 is in transmission connection to the lead screw 300. The sliding block 400 is sleeved on the lead screw 300 and is in transmission connection to the lead screw 300. In this embodiment, the axis direction of the lead screw 300 is consistent with the movement direction of the bolus piston 20, and after the power output shaft outputs power to the transmission component 200, the transmission component 200 transfers the power to the lead screw 300, in order to drive the lead screw 300 to rotate around its own axis, thereby enabling the lead screw 300 to convert the rotational torsion into a linear transmission thrust, pushing the sliding block 400 to move relative to the lead screw 300 along the axis direction of the lead screw 300.

The sliding block 400 is abutted to one end of the push rod 30 away from the bolus piston 20. When the sliding block 400 moves relative to the lead screw 300, it can push the push rod 30 to move in the movement direction of the lead screw 300, thereby pushing the bolus piston 20 to move and achieve the purpose of bolus and drug injection. In addition, in this embodiment, the power output shaft is parallel to the axis of the lead screw 300, and the power output direction of the power output shaft is opposite to the movement direction of the sliding block 400. That is, as shown in Fig. 1, the arrow in Fig. 1 can be understood as the power transmission direction in the power device. In this embodiment, the power transmission path is U-shaped. Therefore, the power device in this embodiment can effectively shorten the length space in the movement direction of the push rod 30 by parallel superposing the power output module 100 and the push rod 30 on the structure, which is conducive to reducing the overall volume of the patch type insulin pump.

In some embodiments, as shown in Fig. 2, the power output module 100, for example, can comprise a stepping motor 110, and, the power device in this embodiment may further comprise a reduction box 500; the reduction box 500 is connected between the power output shaft of the stepping motor 110 and the transmission component 200, that is, the torsion output by the stepping motor 110 can be augmented through reduction by the reduction box 500, then is transferred to the transmission component 200. The reduction box 500 in this embodiment may be a multi-stage planetary gear reduction box, for example, to enable the stepping motor 110 to cooperate with the multi-stage planetary gear reduction box, accurately control the movement speed of the push rod 30, thereby controlling the precision of bolus and drug injection, and improving the user experience.

Continuing with reference to Fig. 2, in some embodiments, the transmission component 200 may comprise at least two gears 210. It can be understood that the transmission component 200 may comprise two gears 210, three gears 210, four gears 210, and so on. All gears 210 can be meshed in order along the arrangement direction of the power output module 100 and the lead screw 300, thereby forming a transmission structure of multi-stage gears 210. Additionally, the reduction box 500 and the lead screw 300 are respectively in transmission connection to two gears 210 at both ends. Taking the three gears 210 in Fig. 2 as an example, the reduction box 500 is in transmission connection to the leftmost gear 210a, and the lead screw 300 is in transmission connection to the rightmost gear 210c. The middle gear 210b is respectively meshed with the gears 210a and 210c. The three gears 210 are located in the same plane to ensure that they move along the axis direction perpendicular to the lead screw 300 when power is transmitted from the gear 210a to the gear 210c, thereby saving the length space in the movement direction of the push rod 30.

Alternatively, in some other embodiments, the transmission component 200 may also be a pulley structure. Specifically, the transmission component 200 may comprise a driving wheel, a driven wheel, and a belt. The driving wheel may be in transmission connection to the reduction box 500, and the driven wheel may be in transmission connection to the lead screw 300; the belt may be wound around the driving wheel and the driven wheel. The power output by the stepping motor 110 can be sequentially transferred to the lead screw 300 through the driving wheel, belt, and driven wheel, thereby driving the lead screw 300 to rotate around its own axis.

Continuing with reference to Figs. 1 and 2, the power device in this embodiment may further comprise a support 600, and the power output module 100, reduction box 500, transmission component 200, and lead screw 300 can all be installed on the support 600 to ensure the stability of the overall structure of the power device. Specifically, the support 600 may comprise a first connecting plate 610, a second connecting plate 620 and a third connecting plate 630, wherein the first connecting plate 610 and the second connecting plate 620 are in parallel and spaced apart, and the third connecting plate 630 is connected between the first connecting plate 610 and the second connecting plate 620, so that the support is U-shaped as a whole.

The stepping motor 110 and the reduction box 500 are both installed on the side surface of the third connecting plate 630 away from the first connecting plate 610. The transmission component 200 is installed on the side surface of the first connecting plate 610 away from the second connecting plate 620, and the gear 210a protrudes from the end of the first connecting plate 610 facing the third connecting plate 630 to facilitate the transmission connection between the gear 210a and the reduction box 500, ensuring that the transmission direction of the power output by the stepping motor 110 is parallel to the movement direction of the bolus piston 20.

Both ends of the lead screw 300 are respectively connected to the first connecting plate 610 and the second connecting plate 620. For example, the first connecting plate 610 and the second connecting plate 620 are respectively provided with connection holes (not shown in the figure). One end of the lead screw 300 passes through the connection hole of the first connecting plate 610 and is in transmission connection to the gear 210c. The other end of the lead screw 300 passes through the connection hole of the second connecting plate 620. The connection holes of the first connecting plate 610 and the second connecting plate 620 can limit the screw 300 in the radial direction of the lead screw 300, ensuring that the lead screw 300 can only rotate relative to the first connecting plate 610 or the second connecting plate 620.

Continuing with reference to Fig. 2, the sliding block 400 is located between the first connecting plate 610 and the second connecting plate 620, and the first connecting plate 610 and the second connecting plate 620 can limit the movement path of the sliding block 400, so that the maximum movement distance of the sliding block 400 is the spacing between the first connecting plate 610 and the second connecting plate 620. In addition, there is also a guide plate 700 connected between the first connecting plate 610 and the second connecting plate 620. Both ends of the guide plate 700 are respectively connected to the first connecting plate 610 and the second connecting plate 620, and the guide plate 700 is parallel to the axis of the lead screw 300. Specifically, there is a certain gap between the guide plate 700 and the lead screw 300 to avoid interference with the rotation of the lead screw 300. The side surface of the sliding bock 400 facing the guide plate 700 can be in contact with the surface of the guide plate 700, so that the guide plate 700 cannot only support the sliding block 400, but also guide the sliding block 400 during movement, ensuring that the sliding block 400 can move along the axis direction of the lead screw 300.

The second connecting plate 620 is also provided with an avoidance hole 621 for avoiding the push rod 30, so that one end of the push rod 30 can pass through the avoidance hole 621 and be abutted to the sliding block 400. The push rod 30 can also be in contact with the end face of the avoidance hole 621 and the surface of the guide plate 700 respectively, so that the combination of the avoidance hole 621 and the guide plate 700 plays a certain guiding role on the push rod 30 when the sliding 400 drives the push rod 30 to move, thereby ensuring that the bolus piston 20 can compress the space inside the cartridge 10 along the predetermined direction.

As an optional embodiment, if the first plane be a plane perpendicular to the axis of the lead screw 300, the forward projection of the cartridge 10 on the first plane can cover the forward projection of the power device on the first plane. That is to say, along the arrangement direction of the stepping motor 110 and the lead screw 300, the size of the cartridge 10 is larger than the size of the power device. Therefore, although the stepping motor 110 and the push rod 30 will be superposed in parallel in this embodiment, the length of the patch type insulin pump on the width of the cartridge 10 will not be increased, thereby effectively reducing the volume of the patch type insulin pump.

Based on the same design concept, the present application may also provide a patch type insulin pump, which can comprise a circuit board, a cartridge 10, a bolus piston 20, a push rod 30, and the power device as described in the above embodiment. The power output module 100 can receive the bolus electrical signal command from the circuit board, and use the power output shaft to output the power to the transmission component 200. The transmission component 200 transfers the power to the lead screw 300, and then drives the sliding block 400 to push the push rod 30 to move, so that the bolus piston 20 compresses the space inside the cartridge 10, ultimately completing the bolus and drug injection.

It will be apparent to those skilled in the art that various amendments and variations may be made to the present application without departing from the spirit and scope of the present application. Therefore, the present application is intended to cover these modifications and variations provided that such modifications and variations made to the present application fall within the scope of the claims of the present application.

## Claims

1. A power device, applied to a patch type insulin pump, wherein the patch type insulin pump comprises a cartridge, a bolus piston and a push rod; the cartridge is used to internally store medicament, and the bolus piston is configured to move relative to the cartridge, in order to compress the space inside the cartridge; the push rod is connected to one side of the bolus piston away from the space inside the cartridge; **characterized in that**, the power device comprises a power output module, a transmission component, a lead screw, and a sliding block;
wherein the power output module comprises a power output shaft, and the power output shaft is in transmission connection to the transmission component;
wherein the transmission component is in transmission connection to the lead screw, and the transmission component is used to transfer power output from the power output shaft to the lead screw to drive the lead screw to rotate around its own axis;
wherein the sliding block is sleeved on the lead screw, and the sliding block is abutted to one end of a push rod away from a bolus piston. When the lead screw rotates around its own axis, the sliding block is driven to move relative to the lead screw along the axis direction of the lead screw, so that the sliding block drives the push rod to move along the axis direction of the lead screw;
wherein, the axis of the power output shaft is parallel to the axis of the lead screw, and the power output direction of the power output shaft is opposite to the movement direction of the sliding block.

2. The power device according to claim 1, **characterized in that**, the power output module comprises a stepping motor.

3. The power device according to claim 2, **characterized by** further comprising a reduction box, the reduction box being connected between the power output shaft and the transmission component.

4. The power device according to claim 1, **characterized in that**, the transmission component comprises at least two gears, and the at least two gears are meshed in order along the arrangement direction of the power output shaft and the lead screw; the reduction box and the lead screw are respectively in transmission connection to the two gears at both ends.

5. The power device according to claim 1, **characterized by** further comprising a support, the power output module, the transmission component and the lead screw being installed on the support.

6. The power device according to claim 5, **characterized in that**, the support comprises a first connecting plate, a second connecting plate and a third connecting plate; wherein the first connecting plate and the second connecting plate are in parallel and spaced apart, and the third connecting plate is connected between the first connecting plate and the second connecting plate, so that the support is U-shaped; and
the power output module is installed on one side of the third connecting plate away from the first connecting plate, and both ends of the lead screw are respectively connected to the first connecting plate and the second connecting plate; the sliding block is located between the first connecting plate and the second connecting plate.

7. The power device according to claim 6, **characterized in that**, the transmission component is installed on one side of the first connecting plate away from the second connecting plate; one end of the lead screw passes through the first connecting plate and in transmission connection to the transmission component; a portion of the transmission component protrudes from one end of the first connecting plate near the third connecting plate.

8. The power device according to claim 6, **characterized by** further comprising a guide plate, wherein the guide plate being connected between the first connecting plate and the second connecting plate, one side of the sliding block being in contact with the guide plate.

9. The power device according to claim 1, **characterized in that**, the forward projection of the cartridge on a first plane covers the forward projection of the power device on the first plane, and the first plane is a plane perpendicular to the axis of the lead screw.

10. A power device for a patch insulin pump to implement bolus and drug injection, **characterized by** comprising the power device according to any of claims 1~9.
